# EUROPÄISCHE PATENTSCHRIFT

(11) **EP 0 816 358 B1**
(45) Veröffentlichungstag und Bekanntmachung des Hinweises auf die Patenterteilung: **03.11.2004**
(21) Anmeldenummer: 97109547.6
(22) Anmeldetag: 12.06.1997
(51) Int. Cl.: C07D 403/04, A61K 31/505

(54) **4-Amino-2-ureido-pyrimidin-5-carbonsäureamide, Verfahren zu deren Herstellung, diese Verbindungen enthaltende Arzneimittel und deren Verwendung**
4-Amino-2-ureido-pyrimidine-5-carboxamides, processes for their preparation, medicaments containing these compounds, and their use
4-Amino-2-uréido-pyrimidine-5-carboxamides, procédés pour leur préparation, médicaments contenant ces composés, et leur application

(30) Priorität: 24.06.1996 DE 19625089
(43) Veröffentlichungstag der Anmeldung: 07.01.1998
(73) Patentinhaber: Aventis Pharma Deutschland GmbH, 65929 Frankfurt am Main (DE)
(72) Erfinder: Böger, Hans Georg, Dr., 65529 Waldems Esch (DE); Hoffmann, Axel, Dr., 65929 Frankfurt (DE); Jähne, Gerhard, Dr., 65929 Frankfurt (DE); Krass, Norbert, Dr., 60487 Frankfurt (DE); Schäfer, Hans-Ludwig, Dr., 55252 Mainz-Kastel (DE)

(56) Entgegenhaltungen:
- EP-A- 0 206 297
- EP-A- 0 557 877
- EP-A- 0 557 879
- EP-A- 0 816 359
- DE-A- 2 853 220

## Beschreibung

Die Erfindung betrifft tertiäre Amide der
4-Amino-2-ureido-pyrimidin-5-carbonsäure sowie deren Säureadditionssalze. Insbesondere betrifft die Erfindung substituierte 4-Amino-2-(imidazolin-2-on-1-yl)-pyrimidin-5-carbonsäure-[N-(fluoralkoxy-substituierte)phenyl-amide] und deren Säureadditionssalze.

Es ist bereits beschrieben worden, 4-Amino-2-ureido-pyrimidin-5-carbonsäure-(Nalkyl-N-phenyl-amide) zur Behandlung von Adipositas und von Lipidstoffwechselstörungen [vgl. Europ. Patentschrift 0 557 879] zu verwenden.

Die Verträglichkeit der in Europ. Patentschrift 0 557 879 als Arzneimittel vorgeschlagenen 4-Amino-2-ureido-pyrimidin-5-carbonsäureamide ist jedoch nicht ganz befriedigend. Diese Stoffe zeigen bei zu hoher Dosierung cytotoxische Effekte. Eine solche Nebenwirkung ist bei einer Verwendung als Therapeutikum unerwünscht.

Der Erfindung lag die Aufgabe zugrunde, Verbindungen zur Verfügung zu stellen, die bei guter Verträglichkeit eine therapeutisch verwertbare hypolipidämische Wirkung entfalten. In diesem Zusammenhang bestand die Aufgabe insbesondere auch darin, Verbindungen zu finden, bei denen neben ausreichender hypolidämischer Wirkung die cytotoxischen Eigenschaften im Vergleich zu den in der Europäischen Patentschrift 0 557 879 beschriebenen Verbindungen nur noch in einem stark reduzierten Maß oder gar nicht mehr zu beobachten sind.

Die Erfindung betrifft daher 4-Amino-2-ureido-pyrimidin-5- carbonsäure-amide der Formel I, 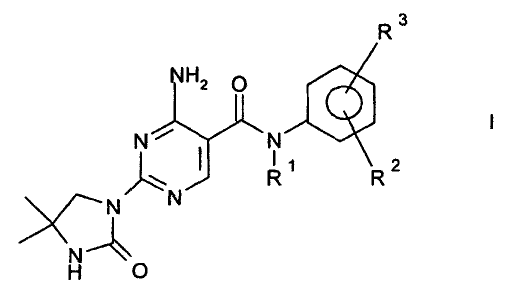 worin bedeuten
- R¹: Wasserstoff;
- R²: -OCF₃;
- R³: H, oder
R² und R³ bilden gemeinsam einen Rest der Formel -O-(C₁-C₅)-alkylen-O-, wobei in dem Alkylenrest alle Wasserstoffe durch Fluor ersetzt sind,
sowie deren physiologisch verträgliche Säureadditionssalze.

Unter physiologisch verträglichen Säureadditionssalzen werden in Wasser leicht lösliche, lösliche und wenig lösliche Verbindungen gemäß der Definition im "Deutschen Arzneibuch" (9. Ausgabe 1986, Amtliche Ausgabe, Deutscher Apotheker-Verlag Stuttgart), Seite 19 verstanden. Bevorzugt sind die Hydrochloride und Sulfate der Verbindungen.

Die Erfindung betrifft weiterhin 2 Verfahren zur Herstellung von 4-Amido-2-ureidopyrimidin-5-carbonsäureamiden der Formel I.

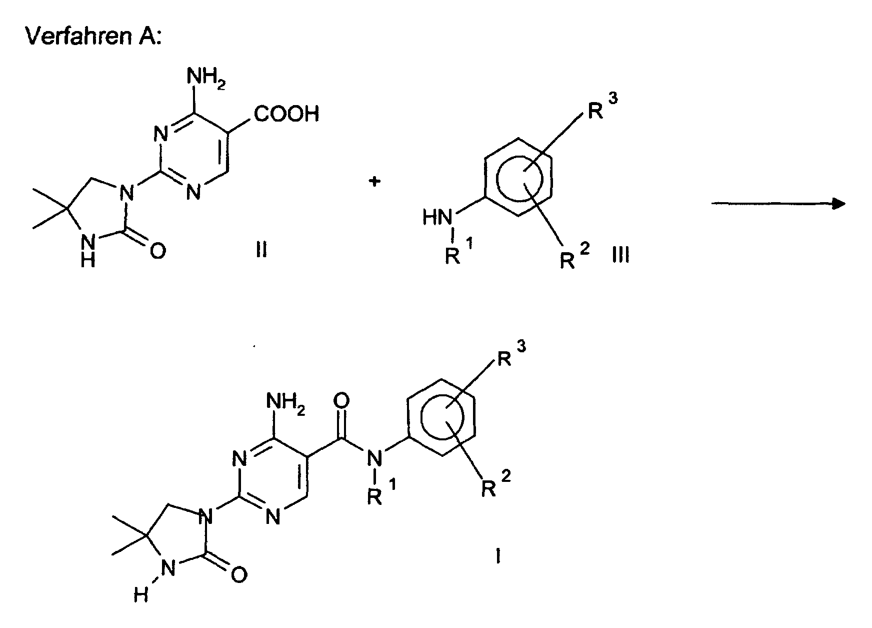

Verfahren A zur Herstellung der Verbindungen der Formel I, dadurch gekennzeichnet, daß man eine Verbindung der Formel II nach einer in situ Aktivierung (Umwandlung in das entsprechende Säurechlorid, beispielsweise mit Thionylchlorid), mit einer Verbindung der Formel III, in der R¹, R² und R³ die zu Formel angegebene Bedeutung haben, bei einer Temperatur von 0 °C bis 200 °C in einem geeigneten Lösungsmittel (wie z. B. DME) mit oder ohne Zusatz einer Hilfsbase (wie z. B. NEt₃) zu einer Verbindung der Formel I umsetzt, und die erhaltene Verbindung der Formel I gegebenenfalls in ein physiologisch verträgliches Salz überführt oder ein erhaltenes Salz gegebenenfalls in ein physiologisch verträgliches Salz überführt.

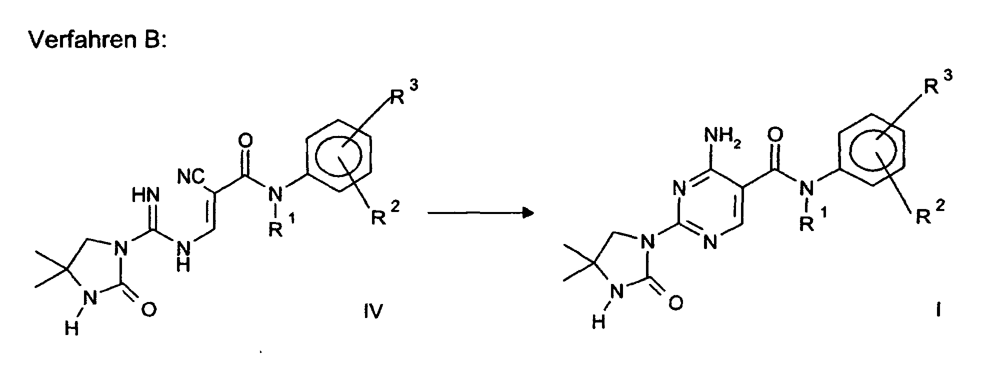

Verfahren B zur Herstellung der Verbindungen der Formel I, dadurch gekennzeichnet, daß man eine Verbindung der Formel IV, in der R¹, R² und R³ die zu Formel I angegebenen Bedeutungen haben, zu einer Verbindung der Formel I cyclisiert. Die Herstellung der Verbindungen vom Typ IV ist, wie auch die Cyclisierung zu Verbindungen von Typ I, sind in EP-0 557 879 beschrieben.

Die 4-Amino-2-(4,4-dimethyl-imidazolin-2-on-1yl)pyrimidin-5-carbonsäure, deren Säurechlorid das Ausgangsprodukt von Verfahren A bildet, wird wie folgt dargestellt:

In der ersten Stufe werden 1-Amidino-4,4-dimethyl-imidazolin-2-on-hydrobromid und 2-Cyano-3-alkoxy-acrylsäurealkylester, bzw. 2-Cyano-3-dimethylaminoacrylsäurealkylester bei einer Temperatur von 0 ° bis 150 °C in einem geeigneten Lösungsmittel, wie z. B. Isopropanol in Gegenwart einer Base, wie z. B. KOH zu 3-{ 1-Amidino-4,4-dimethyl-imidazolin-2-on)-2-cyanoacrylsäurealkylester umgesetzt. In der zweiten Stufe wird 3-(1-Amidino-4,4-dimethyl-imidazolin-2-on)-2-cyanoacryl-säurealkylester bei einer Temperatur von 0 ° bis 150 °C in einem geeigneten Lösungsmittel, wie z. B. Toluol in Gegenwart von Trifluoressigsäure oder Essigsäure zu 4-Amino-2-(4,4-dimethyl-imidazolin-2-on-1-yl)pyrimidin-5-carbonsäurealkylester cyclisiert.

In der dritten Stufe wird der 4-Amino-2-(4,4-dimethyl-imidazolin-2-on-1yl)pyrimidin- 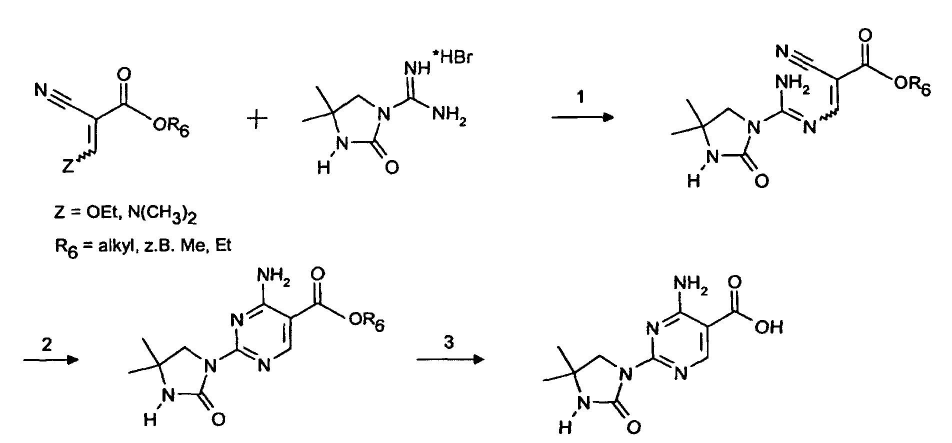 5-carbonsäurealkylester nach bekannten Methoden zur 4-Amino-2-(4,4-dimethylimidazolin-2-on-1-yl)-pyrimidin-5-carbonsäure verseift.

Die vorliegende Erfindung betrifft auch pharmazeutische Zubereitungen, die neben nicht toxischen, inerten, pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten, pharmazeutischen geeigneten Trägerstoffen sind pharmazeutisch unbedenkliche feste, halbfeste oder flüssige Verdünnungsmittel, Füllstoffe und Formulierungshilfsmittel jeder Art zu verstehen, die nach dem Vermischen mit dem Wirkstoff diesen in eine für die Verabreichung geeignete Form bringen.

Als geeignete Anwendungsformen der erfindungsgemäßen Verbindungen kommen beispielsweise Tabletten, Dragees, Kapseln, Pillen, wäßrige Lösungen, Suspensionen und Emulsionen, ggfs. sterile injizierbare Lösungen, nichtwäßrige Emulsionen, Suspensionen und Lösungen, Sprays sowie Zubereitungsförmen mit protrahierter Wirkstoff-Freigabe in Betracht.

Die therapeutisch wirksamen Verbindungen sollen in den oben aufgeführten pharmazeutischen Zubereitungen zweckmäßig in einer Konzentration von etwa 0,1, vorzugsweise von 0,5 bis 99,0, vorzugsweise bis 70,0 Gewichtsprozenten der Gesamtmischung vorhanden sein.

Die Anwendungskonzentrationen für Lösungen sowie Aerosole in Form von Spray betragen im allgemeinen 0,1 bis 20, vorzugsweise 0,5 - 5 Gewichtsprozent.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe(s) mit dem oder den Trägerstoff(en).

Die Wirkstoffe oder die pharmazeutischen Zubereitungen können oral, parenteral, intraperitoneal und/oder rectal appliziert werden.

Die z. B. als Hypolipidämika verwendbaren Verbindungen der vorliegenden Erfindung, und ihre Salze, können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Kapseln (Gelatinekapseln), welche den Wirkstoff zusammen mit Verdünnungsmitteln bzw. Trägerstoffen, z. B. Lactose, Dextrose, Rohrzucker, Mannitol, Sorbitol, Cellulose, verschiedenen Stärkearten und/oder Glycin, und Gleitmitteln wie Kieselerde, Talk, Stearinsäure oder deren Salze, wie Magnesiumoder Calciumstearat, und/oder Polyethylenglykol enthalten. Tabletten enthalten ebenfalls Bindemittel wie Magnesiumcarbonat, Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wässrige Lösungen oder Suspensionen, die sterilisiert sein können und Hilfsstoffe, wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die wenn erwünscht weitere pharmakologisch wirksame Stoffe enthalten können, werden z. B. mittels konventioneller Misch-Granulier- und Dragierverfahren, hergestellt und enthalten 0,1 % bis vorzugsweise 80 %, bevorzugt etwa 5 % bis etwa 65 %, des Wirkstoffs.

Die orale Anwendung erfolgt in pharmazeutisch üblichen Zubereitungen, beispielsweise in Form von Tabletten, Dragees oder Kapseln, die z. B. pro Tagesdosis 5 bis 1000 mg, vorzugsweise 20 bis 200 mg, des Wirkstoffes in Mischung mit einem gebräuchlichen Trägerstoff und/oder Konstituents enthalten, wobei Einzeldosen von 5 bis 200 mg, vorzugsweise ein- bis dreimal täglich, gegeben werden können.

Es kann jedoch erforderlich sein, von den genannten Dosierungen abzuweichen und zwar in Abhängigkeit von der Art und dem Körpergewicht des zu behandelnden Objekts, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen

Dosierung und Applikationsart der Wirkstoffe kann durch jeden Fachmann aufgrund seines Fachwissens leicht erfolgen.

Die Verbindungen der Formel I und deren physiologisch verträglichen Salze stellen durch ihre geringe Cytotoxizität ideale Arzneimittel zur Behandlung von Lipidstoffwechselstörungen, insbesondere von Hyperlipidämie dar. Die Verbindungen sind besonders geeignet durch Stimulierung des LDL-Rezeptors, die Plasmaspiegel wirkungsvoll zu senken. Folgende Befunde belegen die pharmakologische Wirksamkeit der beschriebenen Verbindungen

### 1. In Rattenlebern werden innerhalb weniger Stunden die LDL-Rezeptor mRNA Level durch Verbindungen der Formel I gesteigert (Tabelle I).

Die Stimulierung liegt im Bereich von 170 bis 350 % der Kontrollen (Kontrolle = 100 %).

Die Präparation der mRNA wurde nach der Methode von Chomczynski, P. und Sacchi, N., Anal. Biochem. 162, 156 - 159 (1987) durchgeführt. Bei Organen (wie z. B. Leber) wurde das tiefgefrorene Gewebe auf Trockeneis zuvor im Mörser homogenisiert und die mRNA mittels Oligo dT nach Standardmethoden weiter angereichert (vgl. Sambrook, J., Fritsch, E. F. und Maniatis, T., Molecular Cloning, zweite Auflage, Cold Spring Harbor (1989); in dieser Methodensammlung finden sich auch Beschreibungen aller weiteren hier verwendeten relevanten molekularbiologischen Standardverfahren). 5 bis 20 *µ*m der so gewonnenen gelösten mRNA wurden nach Standardverfahren denaturiert und auf 1 %igen horizontalen Agarosegelen aufgetrennt. Die mRNA wurde mittels Kapillarblot auf Hybond N Membranen (Amersham) transferiert. Als spezifische Hybridisierungsprobe diente ein partieller LDL-Rezeptor cDNA Klon und als interner Standard ein Plasmid, das ein β-Aktin Gen enthielt. Beide Plasmide wurden mittels eines random Primer Kit von Amersham bis zu einer spezifischen Aktivität von 5 x 10⁹ cpm/*µ*g markiert. Vorhybridisierung, Hybridisierung und Waschen der Filter erfolgten nach Standardverfahren. Die Filter wurden anschließend auf Cronex 4 Filmen (Dupont) über Nacht bis zu 14 Tagen in Gegenwart einer Verstärkerfolie bei -70°C exponiert und die Hybridisierungssignale über die Filmschwärzungsintensität mit einem handelsüblichen Laserdensiometer quantifiziert. Anschließend wurde der Quotient aus der Intensität der LDL-Rezeptorbande und der Aktinbande als internem Standard zur Korrektur von Ausbeutevariationen bestimmt.

Tabelle I zeigt die Stimulierung der LDL-Rezeptor-mRNA-Expression in Rattenlebem 6 Stunden nach einer Applikation ausgewählter Verbindungen der Formel I (Dosis von 30 mg/kg). Lebergewebe wurde entnommen und in flüssigem Stickstoff schockgefroren.

Anschließend wurde die mRNA wie beschrieben isoliert und mittels der Northem-Blot-Technik die relativen LDL-Rezeptor-mRNA-Level bestimmt.

mRNA Level von unbehandelten Kontrolltieren wurden als 100 % gesetzt und die Stimulierung der LDL-Rezeptor-mRNA in Prozent der Kontrolle errechnet.

**Tabelle I**

| Verbindungen gemäß Beispiel | Konzentr. | LDL-Rezeptor-mRNA |
|---|---|---|
| 2 | 30 mg/kg | 250% |
| 14 | 30 mg/kg | 250% |
| 15 | 30 mg/kg | 242% |

### 2. Vergleichende antiproliferative Untersuchungen:

Exponentiell wachsende Tumorzellen (Bronchialkarzinomzellen, A549, Kolononkarzinomzellen, HT29, Nierenkarzinom-Zellen, Helazellen) werden in einer Konzentration von 5 x 10³ Zellen pro ml in RPMI Standard Medium in 96 Loch Mikrotiterplatten ausgesäht. Die Inkubation mit Testsubstanz-Konzentrationsreihen erfolgt 72 Stunden bei 37 °C, 5 % CO₂, 95 % rel. Luftfeuchtigkeit. Jede Verbindungskonzentration bzw. Kontrolle wird dabei in vier Parallelinkubationen getestet. Nach 65 Stunden werden 50 µl MTT [3-(4,5-Dimethyl-2-thiazolyl)-2,5-diphenyl-2H-tetrazolium-bromid] in 2,5 mg/ml PBS zugesetzt. In intakten Zellen wird MTT reduziert zu einem roten unlöslichen Farbstoff. In Abhängigkeit von der verwendeten Zellinie wird nach weiteren 7 bis 24 Stunden Inkubation der Überstand entfernt. Der entstandene unlösliche Farbstoff wird in 100 µl DMSO unter vorsichtigem Schütteln gelöst und die Extinktion in einem Multiscan Photometer 340 CC der Fa. Flow bei 492 nm gemessen.

Die Ergebnisse werden als Quotienten aus den gemittelten Extinktionswerten der Testsubstanzen und der Kontrollwerte berechnet. Die Schwankungen der Einzelbestimmungswerte innerhalb der Parallelwerte sind kleiner als 15 %. Aus Dosis-Wirkungsdiagrammen wird für die angegebenen Verbindungen der IC₅₀ Wert abgelesen.

Als Vergleichsverbindung A wurde 4-Amino-2-(4,4-dimethyl-2-oxo-imidazolin-1-yl)-pyrimidin-5-carbonsäure-(N-ethyl-N-3-trifluormethyl-phenyl)-amid)-hydrochlorid (Verbindung aus Beispiel 2 aus EP 0557 879) getestet.

**Tabelle II**

| MTT-ASSAY IC₅₀[µg/ml] | | | |
|---|---|---|---|
| Beispiel | HeLa | HT 29 | A 549 |
| Vergleichsverbindung A | 9,6 | 13 | 12,4 |
| 2 | >100 | >100 | >100 |
| 14 | >100 | >100 | n.b. |
| 15 | >100 | >100 | n.b. |

Aus Tabelle II ist abzulesen, daß die antiproliferativen Eigenschaften und somit die Cytotoxizität der erfindungsgemäßen Verbindungen der Formel I im Gegensatz zu denen der Vergleichsverbindung A stark herabgesetzt sind.

Die nachfolgenden Beispiele dienen zur näheren Erläuterung der Erfindung, ohne dieselbe auf in den Beispielen beschriebene Produkte und Ausführungsformen einzuschränken.

### Beispiel 2 (Methode B):

4-Amino-2-(4,4-dimethyl-imidazolin-2-on-1-yl)-5-(3-trifluormethoxy-phenylpyrimidin-carbonsäureamid

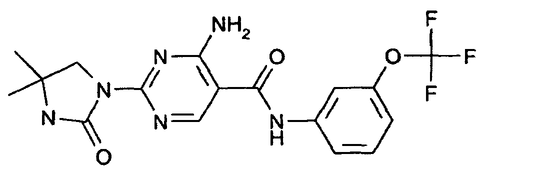

### Stufe 1 :

2.0 g (11.3 mmol) 3-Trifluormethoxy-anilin werden unter Zusatz von 2.35 ml (17 mmol) Triethylamin in 7 ml trockenem Acetonitril gelöst. Man kühlt auf 0°C und setzt portionsweise 0.96 g (11.3 mmol) Cyanessigsäure zu. Nach 5 min bei -10°C wird eine Lösung von 0.5 ml (5.65 mmol) Phosphortrichlorid in 1.7 ml Acetonitril zugetropft. Es wird 4.5 h bei Raumtemperatur nachgerührt, Wasser zugesetzt und mit Essigsäureethylester extrahiert. Waschen der vereinigten organischen Phasen mit ges. NaCl-Lösg., trocknen über Magnesiumsulfat und einengen liefert 2.8 g (= 100%) Cyanessigsäure-(3-trifluormethoxy)-anilid.

Smp.: 115-117°C; MS: m/e 245 (M⁺ +1); 270 MHz ¹H-NMR (CDCl₃, ppm): 3.60 (s, 2H), 7.0-7.1 (brs, 1H), 7.40 (m, 2H), 7.55 (brs, 1H), 7.85 (brs,1H).

### Stufe 2:

2.8 g (11.3 mmol) Cyanessigsäure-(3-trifluormethoxy)-anilid werden mit 8.3 ml N,N-Dimethylformamid-dimethylacetal (62 mmol)2 h bei Raumtemperatur gerührt. Nach Stehen über Nacht erfolgt säulenchromatographische Reinigung mit Essigsäureethylester / n-Heptan 1:1 als Eluent. Isolierung von 2.8 g 2-Cyano-3-N,N-dimethylamino-acrylsäure-(3-trifluor-methoxyphenyl)-amid (= 100%).

Smp.: 128°C; MS: m/e 300.2 (M⁺ +1); 270 MHz ¹H-NMR (CDCl₃, ppm): 3.25 (s, 3H), 3.40 (s, 3H), 6.95 (m, 1H), 7.35 (m, 2H), 7.70 (m, 2H), 7.90 (s, 1H).

### Stufe 3:

2.7g (9.0 mmol) 2-Cyano-3-N,N-dimethylamino-acrylsäure-(3-trifluormethoxyphenyl)-amid und 1.5g (9.9 mmol) 1-Amidino-4,4-dimethyl-2-oxoimidazolidin werden in 100 ml trockenem Ethanol 6 Stunden am Rückfluß gekocht. Man läßt abkühlen engt bis zur Trockene ein und nimmt mit wenig Essigsäureethylester auf. Abfiltrieren, Nachwaschen mit wenig kaltem Essigsäureethylester und Trocknung im Vakuum bei 45°C liefert 2.0g 4-Amino-2-(4,4-dimethyl-imidazolin-2-on-1-yl)-5[(3-trifluormethoxy)-phenyl]-pyrimidincarbonsäureamid. Dies entspricht einer Ausbeute von 54%.

Smp.: 295-297°C (Zers.); MS: m/e 411.2 (M⁺ +1); 200 MHz ¹H-NMR (DMSO-d₆, ppm): 1.30 (s, 6H), 3.70 (s, 2H), 7.10 (m, 1H), 7.4 (s, 1H), 7.45 (dd, 1H). 7.65 (brs, 3H), 7.8 (m, 1H), 8.7 (s, 1H), 10.3 (s, 1H).

### Beispiel 9:

4-Amino-2-(4,4-dimethyl-2-imidazolin-2-on-1-yl)-5-[N-(2-trifluormethoxy-phenyl)]-pyrimidincarbonsäureamid-Hydrochlorid

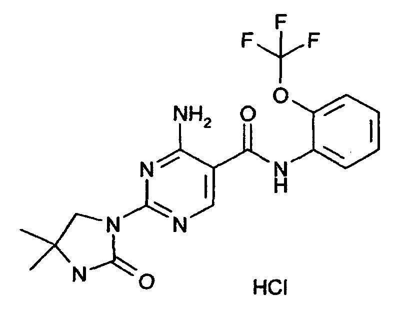

Ausbeute: 33%. Smp.: 295°C. MS: m/e 411.3 (M⁺ +1); 270 MHz ¹H-NMR (DMSOd₆, ppm): 1.35 (s, 6H), 3.75 (s, 2H), 7.35-7.50 (m, 3H), 7.65 (m, 1H), 8.65 (s,1H), 8.70 (s, 1H), 8.75-9.40 (brs, 2H), 10.90 (s, 1H).

### Beispiel 10:

4-Amino-2-(4,4-dimethyl-imidazolin-2-on-1-yl)-5-[N-(4-trifluormethoxy-phenyl)]-pyrimidincarbonsäureamid-Hydrochlorid

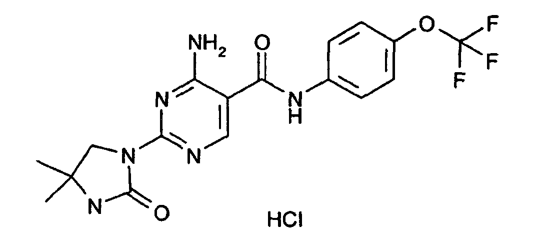

Ausbeute: 18%. Smp.: >300°C. MS: m/e 411.2 (M⁺ +1); 200 MHz ¹H-NMR (DMSO-d₆, ppm): 1.35 (s, 6H), 3.65 (s, 2H), 7.40 (d, 2H), 7.85 (d, 2H), 8.65 (d, 2H), 8.80 (brs, 1H), 9.30 (brs, 1H), 11.15 (s, 1H).

### Beispiel 14:

4-Amino-2-(4,4-di methyl-imidazolin-2-on-1-yl)5-[N-(2,2-difluor-benzo-1,3-dioxol-5-yl)]-pyrimidincarbonsäureamid-Hydrochlorid.

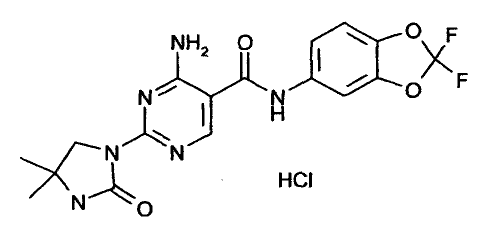

Ausbeute: 81 %. Smp.: 318°C; MS: m/e 407 (M⁺+1); 200 MHz ¹H-NMR (DMSOd₆, ppm): 1.35 (s, 6H), 3.75 (s, 2H), 7.45 (d, 1H), 7.55 (dd, 1H), 7.90 (d, 1H), 8.75 (s, 1H), 8.85 (brs, 1H), 9.35 (brs, 1H), 11.40 (s, 1H).

### Beispiel 15:

4-Amino-2-(4,4-dimethyl-imidazolin-2-on-1-yl)-5-[N-(2,2,3,3-tetrafluorbenzo-1,4-dioxan-6-yl)]-pyrimidincarbonsäureamid-Hydrochlorid.

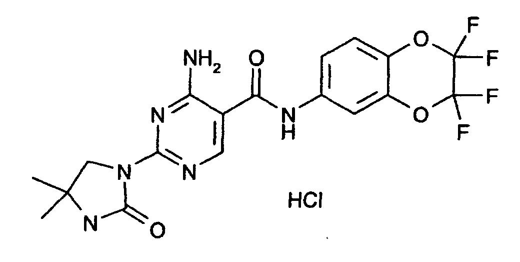

Ausbeute: 96 %. Smp.: 315°C; MS: m/e 557 (M⁺+1); 200 MHz ¹H-NMR (DMSOd₆, ppm): 1.35 (s, 6H), 3.75 (s, 2H), 7.50 (d, 1H), 7.65 (dd, 1H), 7.90 (d, 1H), 8.65 (s, 1H), 8.70 (s, 1H), 8.80 (brs, 1H), 9.25 (brs, 1H), 11.40 (s, 1H).

Die in der Beschreibung verwendeten Abkürzungen haben folgende Bedeutungen:
- DME: Dimethoxyethan
- NEt₃: Triethylamin
- LDL: low-density-lipoprotein
- h: Stunde
- NMP: N-Methyl-pyrrolidon
- DMF: Dimethylformamid

## Patentansprüche

1. 4-Amino-2-ureido-pyrimidin-5-carbonsäure-amide der Formel I, 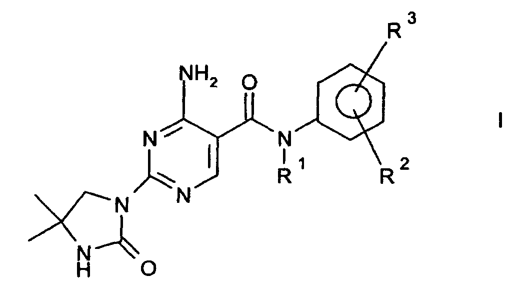 worin bedeuten
R¹ Wasserstoff;
R² -OCF₃;
R³ H; oder
R² und R³ bilden gemeinsam einen Rest der Formel -O-(C₁-C₅)-alkylen-O-, wobei in dem Alkylenrest alle Wasserstoffe durch Fluor ersetzt sind,
sowie deren physiologisch verträgliche Säureadditionssalze.

2. Verfahren zur Herstellung von Verbindungen der Formel I, gemäß Anspruch 1 **dadurch gekennzeichnet, daß** man nach folgendem Formelschema 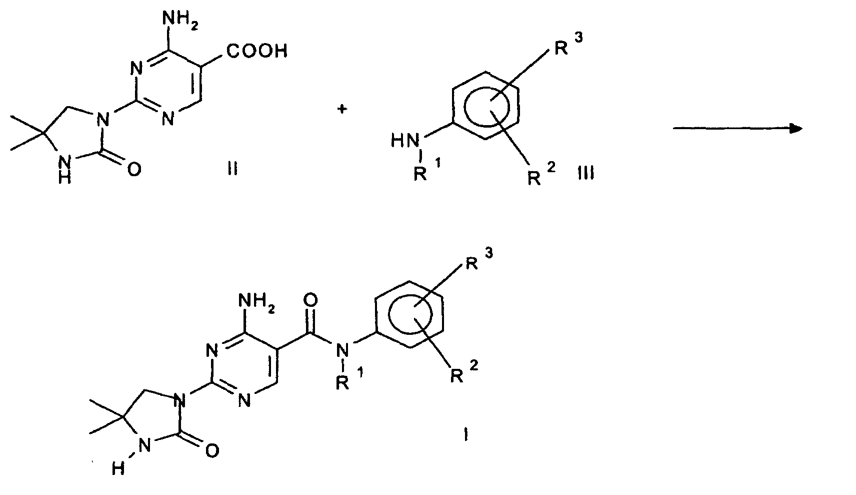 eine Verbindung der Formel II in das entsprechende Säurechlorid überführt und dieses mit einer Verbindung der Formel IIII, in der R¹, R² und R³ die zu Formel I angegebenen Bedeutungen haben, bei einer Temperatur von 0°C bis 200°C in einem geeigneten Lösungsmittel mit oder ohne Zusatz einer Hilfsbase zu einer Verbindung der Formel I umsetzt und die erhaltene Verbindung der Formel I gegebenenfalls in ein physiologisch verträgliches Salz überführt oder ein erhaltenes Salz in ein physiologisch verträgliches Salz überführt.

3. Verfahren zur Herstellung von Verbindungen der Formel I, gemäß Anspruch 1, **dadurch gekennzeichnet, daß** man nach folgendem Formelschema 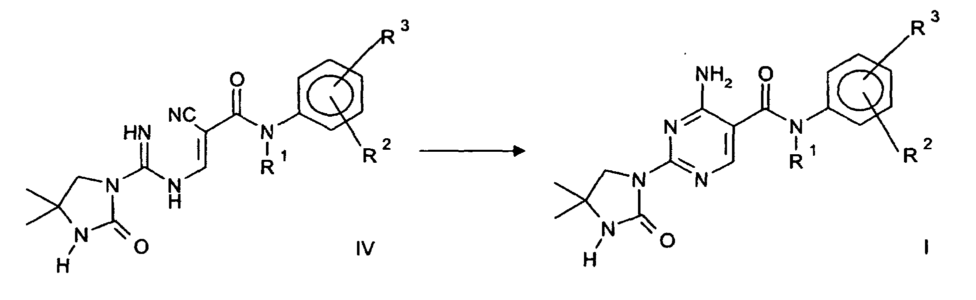 eine Verbindung der Formel IV, in der R1, R2 und R3 die zu Formel I angegebene Bedeutung haben, zu einer Verbindung der Formel I cyclisiert.

4. Arzneimittel enthaltend eine oder mehrere Verbindung(en) gemäß Anspruch 1.

5. Verbindungen gemäß Anspruch 1 zur Behandlung von Lipidstoffwechselstörungen.

6. Verbindungen gemäß Anspruch 1 zur Behandlung von Hyperlipidämie.

## Claims

1. A 4-amino-2-ureidopyrimidine-5-carboxamide of the formula I, 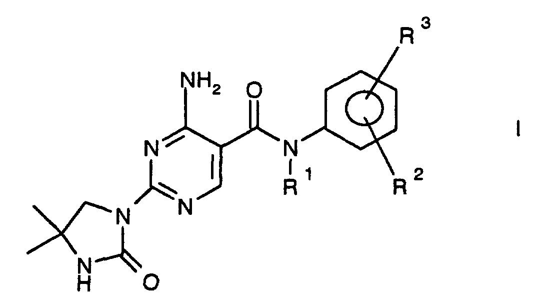 in which
R¹ is hydrogen,
R² is -OCF₃,
R³ is H, or
R² and R³ together form a radical of the formula -O-(C₁-C₅)-alkylen-O-, where in the alkylene radical all hydrogens are replaced by fluorine,
or its physiologically tolerable acid addition salts.

2. A process for the preparation of compounds of the formula I, as claimed in claim 1, which comprises, according to the following reaction scheme 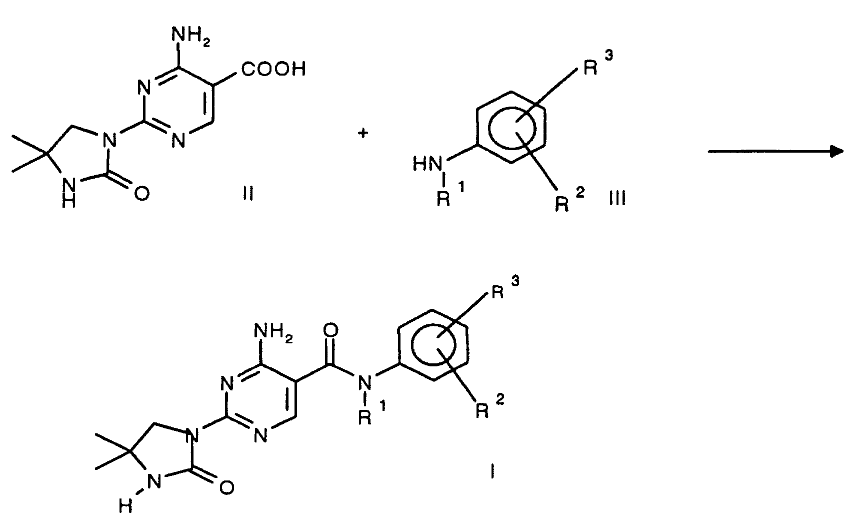 converting a compound of the formula II into the corresponding acid chloride and reacting this with a compound of the formula III, in which R¹, R² and R³ have the meanings indicated for formula I, at a temperature from 0°C to 200°C in a suitable solvent with or without addition of an auxiliary base to give a compound of the formula I and optionally converting the compound of the formula I obtained into a physiologically tolerable salt or converting a salt obtained into a physiologically tolerable salt.

3. A process for the preparation of compounds of the formula I as claimed in claim 1, which comprises, according to the following reaction scheme 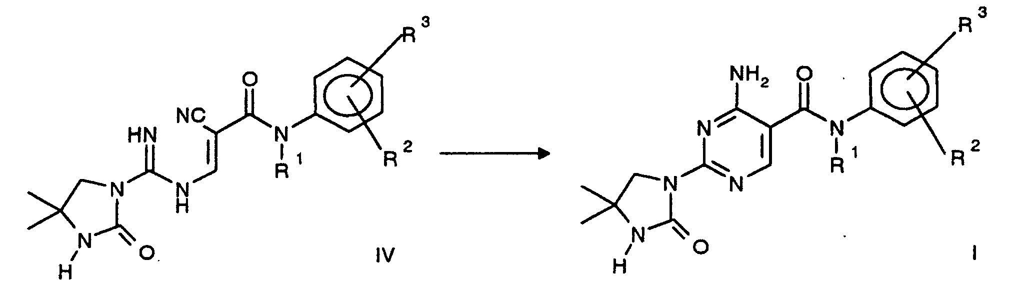 cyclizing a compound of the formula IV, in which R¹, R² and R³ have the meanings indicated for formula I, to a compound of the formula I.

4. A pharmaceutical comprising one or more compounds as claimed in claim 1.

5. A compound as claimed in claim 1 for the treatment of disorders of lipid metabolism.

6. A compound as claimed in claim 1 for the treatment of hyperlipidemia.

## Revendications

1. Amides de l'acide 4-amino-2-uréidopyrimidine-5-carboxylique de formule I, 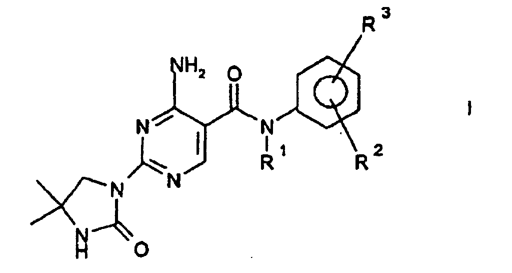 dans laquelle
R¹ signifie hydrogène ;
R² signifie -OCF₃ ;
R³ signifie H ; ou
R² et R³ forment ensemble un radical de formule -O-(alkylène en C₁ à C₅)-O-, tous les atomes d'hydrogène dans le radical alkylène étant remplacés par fluor, ainsi que leurs sels d'addition d'acide physiologiquement acceptables.

2. Procédé pour la préparation de composés de formule I, selon la revendication 1, **caractérisé en ce qu'**on transforme, selon le schéma de formules suivant, 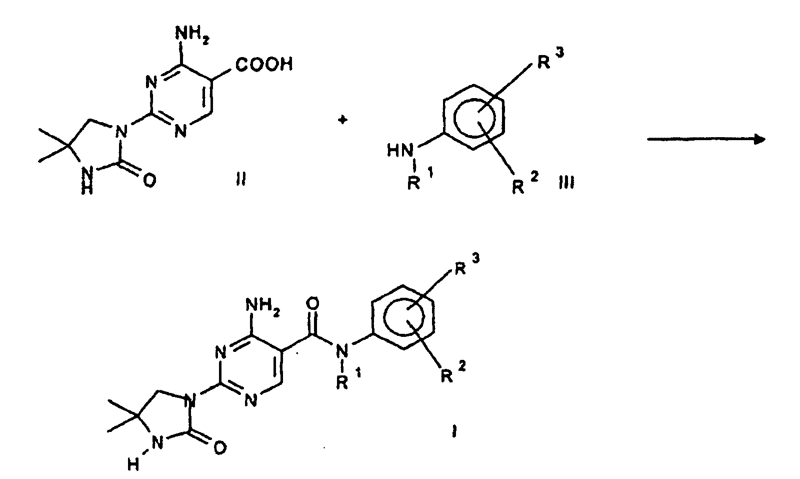 un composé de formule II en chlorure d'acide correspondant et on transforme celui-ci avec un composé de formule III, dans laquelle R¹, R² et R³ ont les significations indiquées pour la formule I, à une température de 0°C à 200°C, dans un solvant approprié, avec ou sans addition d'une base auxiliaire, en composé de formule I et on transforme le composé de formule I obtenu le cas échéant en un sel physiologiquement acceptable ou on transforme un sel obtenu en un sel physiologiquement acceptable.

3. Procédé pour la préparation de composés de formule I, selon la revendication 1, **caractérisé en ce qu'**on cyclise, selon le schéma de formules suivant 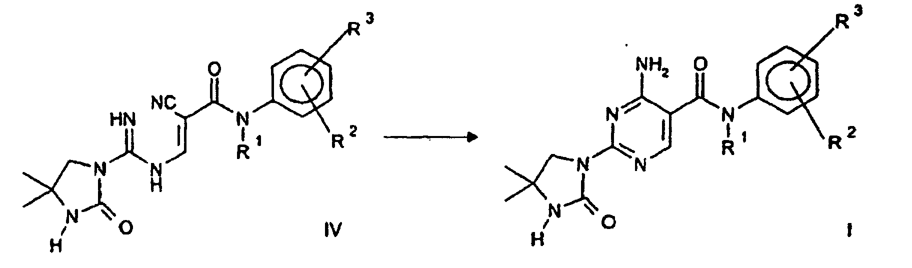 un composé de formule IV, dans lequel R¹, R² et R³ ont la signification indiquée pour la formule I, en un composé de formule I.

4. Médicament, contenant un ou plusieurs composés selon la revendication 1.

5. Composés selon la revendication 1 pour le traitement de troubles du métabolisme des lipides.

6. Composés selon la revendication 1 pour le traitement de l'hyperlipidémie.
